# EUROPEAN PATENT APPLICATION

(11) **EP 1 470 817 A1**
(43) Date of publication of application: **27.10.2004**
(21) Application number: 02806665.2
(22) Date of filing: 20.09.2002
(51) Int. Cl.: A61K 31/355, A61K 31/01, A61K 31/015, A61K 31/047, A61K 31/12, A61K 31/192, A61K 31/352, A61K 31/353, A61K 31/375, A61K 31/525, A61K 31/7048, A61P 35/00

(54) **COMPOSITIONS FOR PREVENTING HUMAN CANCER AND METHOD OF PREVENTING HUMAN CANCER**

(30) Priority: 31.01.2002 JP 2002022958
(71) Applicant: Kansai Technology Licensing Organization Co., Ltd., Kyoto-shi, Kyoto 600-8815 (JP)
(72) Inventor: NISHINO, Hoyoku, Hirakata-shi, Osaka 573-1144 (JP); JINNO, Kenji, Matsuyama-shi, Ehime 790-0854 (JP)
(74) Representative: Müller-Boré & Partner Patentanwälte
(86) International application number: PCT/JP2002/009700
(87) International publication number: WO 2003/063860

(57) **Abstract**

The composition of the present invention comprises vitamin E compounds in addition to carotenoid compounds. Carotenoid compounds and vitamin E compounds are preferably administered according to the method of the present invention such that the daily dosage of carotenoid compounds is 1-100 mg, and the daily dosage of vitamin E compounds is 10-200 mg. When capsules comprising 10 mg natural lycopene, 6 mg natural β-carotene, 3 mg natural α-carotene, 1 mg other natural carotenoids, and α-tocopherol were administered to hepatic cirrhosis patients over a period of 5 years, the incidence rate of hepatic cancer after 5 years in the group who took the capsules was one-third that of the group who did not take the capsules. Significant cancer-preventive effects in humans are manifested for the first time in the composition of the present invention.

## Description

### TECHNICAL FIELD

The present invention relates to a composition used in the prevention of cancer in humans, and to a method of human cancer prevention using the same. More particularly, the present invention relates to a composition appropriate for patients of hepatic diseases which tend to progress toward hepatic cancer, such as chronic hepatitis or hepatic cirrhosis, and to a method of human cancer prevention.

### BACKGROUND ART

Cancer is the leading cause of death, and it is predicted that cancer deaths will continue to increase in the future. While various methods exist for fighting cancer, prevention has received attention in recent years. The search for chemical compounds and food products having cancer-preventive effects is therefore being carried out by means of cellular experimentation, animal experimentation, and the like.

However, chemical compounds and food products for which cancer-preventive effects have been proven in humans have not heretofore existed. For example, β-carotene is thought to be a chemical compound useful in preventing cancer; however, it is reported that when β-carotene alone is administered in large quantities to humans, the incidence rate of pulmonary cancer actually rises (Omenn, G.S., Goodman, G.E., Thornquist, M.D., Balmes, J., Cullen, M.R., Glass, A., Keogh, J.P., Meyskens, F.L., Jr., Valanis, B., Williams, J.H., Jr., Barnhart. S. and Hammar, S., Effect of a combination of beta carotene and vitamin A on lung cancer and cardiovascular disease. *New Engl*. *J. Med.*, 334, 1150-1155, 1996.).

### DISCLOSURE OF THE INVENTION

An object of the present invention is to provide a composition having human cancer-preventive effects and to provide an effective method of human cancer prevention.

The inventors discovered the following information after extensive research aimed at achieving the aforementioned objects.
i) As previously mentioned, it is reported that the administration of large quantities of a single carotenoid compound by itself to humans may give rise to cancer. With the foregoing in view, the incidence of cancer can be prevented if multiple carotenoid compounds in combination are administered to humans in doses that are smaller than single carotenoid doses that cause cancer, and if vitamin E compounds are administered together with multiple carotenoid compounds. Particularly, the progression of hepatic diseases towards hepatic cancer can be effectively prevented. General malaise in patients of hepatic diseases can also be alleviated.
ii) Even more exceptional cancer-preventive effects can be obtained by administering a plurality of types of carotenoid compounds.

The present invention, which was completed on the basis of the aforementioned information, comprises the composition and method of human cancer prevention according to the following items.
1. A composition comprising carotenoid compounds and vitamin E compounds.
2. The composition according to item 1, comprising at least two types of carotenoid compounds.
3. The composition according to item 1, comprising lycopene and carotenes as carotenoid compounds.
4. The composition according to item 1, comprising 150-5000 parts by weight of vitamin E compounds per 100 parts by weight of carotenoid compounds.
5. The composition according to item 1, comprising 10 parts by weight of lycopene, 3-6 parts by weight of β-carotene, 1-3 parts by weight of α-carotene, 1-3 parts by weight of phytoene, and 30-100 parts by weight of vitamin E compounds.
6. The composition according to item 5, comprising 10 parts by weight of lycopene, 6 parts by weight of β-carotene, 3 parts by weight of α-carotene, 1 part by weight of phytoene, and 50 parts by weight of vitamin E compounds.
7. The composition according to item 1, further comprising flavonoid compounds.
8. The composition according to item 1, further comprising ascorbic acid compounds.
9. The composition according to item 1, further comprising at least one antioxidant compound selected from a group consisting of vitamin B2 tetrabutyrate, anthocyanin compounds, catechin, curcumin, inositol, and ferulic acid.
10. The composition according to item 1, comprising carotenoid compounds and vitamin E compounds such that a daily dosage consists of 1-100 mg of carotenoid compounds and 10-200 mg of vitamin E compounds.
11. The composition according to item 1, in the form of a pharmaceutical preparation.
12. The composition according to item 1, further comprising food products.
13. A method of human cancer prevention for preventing cancer in humans by means of administering a composition that comprises carotenoid compounds and vitamin E compounds.
14. The method of human cancer prevention according to item 13, wherein the composition comprises at least two types of carotenoid compounds.
15. The method of human cancer prevention according to item 13, wherein the composition comprises lycopene and carotenes as carotenoid compounds.
16. The method of human cancer prevention according to item 13, wherein the composition comprises 150-5000 parts by weight of vitamin E compounds per 100 parts by weight of carotenoid compounds.
17. The method of human cancer prevention according to item 13, wherein the composition comprises 10 parts by weight of lycopene, 3-6 parts by weight of β-carotene, 1-3 parts by weight of α-carotene, 1-3 parts by weight of phytoene, and 30-100 parts by weight of vitamin E compounds.
18. The method of human cancer prevention according to item 17, wherein the composition comprises 10 parts by weight of lycopene, 6 parts by weight of β-carotene, 3 parts by weight of α-carotene, 1 part by weight of phytoene, and 50 parts by weight of vitamin E compounds.
19. The method of human cancer prevention according to item 13, wherein the composition further comprises flavonoid compounds.
20. The method of human cancer prevention according to item 13, wherein the composition further comprises ascorbic acid compounds.
21. The method of human cancer prevention according to item 13, wherein the composition further comprises at least one antioxidant compound selected from a group consisting of vitamin B2 tetrabutyrate, anthocyanin compounds, catechin, curcumin, inositol, and ferulic acid.
22. The method of human cancer prevention according to item 13, wherein the composition is in the form of a pharmaceutical preparation.
23. The method of human cancer prevention according to item 13, wherein the composition further comprises food products.
24. The method of human cancer prevention according to item 13, wherein 1-100 mg a day of carotenoid compounds and 10-200 mg a day of vitamin E compounds are administered.
25. The method of human cancer prevention according to item 24, wherein 5-15 mg a day of lycopene, 1-10 mg a day of β-carotene, 1-10 mg a day of α-carotene, 1-10 mg a day of phytoene, and 10-200 mg a day of vitamin E compounds are administered.
26. The method of human cancer prevention according to item 13, wherein the daily dosage of a single carotenoid compound is no more than 20 mg.
27. The method of human cancer prevention according to item 13 for preventing the progression towards hepatic cancer from hepatic diseases in human.

A composition having cancer-preventive effects in humans and a method of effectively preventing human cancer are provided by means of the present invention described above.

More particularly, cancer-preventive effects in humans are manifested for the first time in the composition of the present invention. Therefore, the progression towards cancer is suppressed when the composition of the present invention is administered in humans, especially for people who are suffering from diseases that can progress towards cancer. Particularly, the progression towards hepatic cancer in patients of hepatic diseases for which there is a potential for progression towards hepatic cancer (chronic hepatitis, hepatic cirrhosis, and the like) is effectively suppressed.

Also, general malaise is alleviated and QOL (Quality of Life) is improved when the composition of the present invention is administered to such patients.

Furthermore, the potential for causing pulmonary cancer also exists as the single-compound daily dosage of carotenoid compounds begins to exceed 20 mg. Because the composition of the present invention comprises a plurality of these compounds, cancer prevention aimed at minimizing the induction of pulmonary cancer is possible in the method of the present invention when the daily dosage of each compound in the composition of the present invention is kept below 20 mg.

It is difficult to precisely administer the necessary quantities of each chemical compound through a food product because of variations in the ingredients of individual food products within otherwise similar types of food products. In this aspect, the danger of pulmonary cancer or the like arising from excessive dosage is easily avoided using the composition of the present invention, because the dosage of each compound is thus simpler to control than when administration is carried out through food products.

Furthermore, when the cancer-preventing compounds contained in the composition of the present invention are administered using food products, specific food products must be consumed in large quantities, effort is required to administer the necessary quantities, and it is difficult to take the necessary quantities. However, the necessary quantities of each chemical compound can be easily administered by means of the composition of the present invention. It is difficult to precisely administer the necessary quantities of each compound through food product because of variations in the ingredients of individual food products within otherwise similar type of food products. However it is easy to control the administration dosage of each compound with the composition of the present invention. Furthermore it is also easy to control the ratio of the dosage of each compound. When certain compounds in the composition of the present invention are administered from food products, the administered quantity of each ingredient can be easily controlled by dispensing with the administration of the corresponding compounds.

The composition of the present invention having the effects described above may be used in the form of pharmaceutical preparations or food products. Namely, human cancer can be effectively prevented by administration of pharmaceutical embodiments or food product embodiments to humans, in addition to embodiments involving active ingredients alone.

### DETAILED DESCRIPTION OF THE INVENTION

### (I) Composition

### Summary

The composition of the present invention comprises carotenoid compounds and vitamin E compounds. The composition of the present invention is capable of effectively preventing human cancer, and hepatic cancer in particular. Namely, the composition is particularly suited for preventing the progression towards hepatic cancer from hepatic diseases with a high probability of hepatic cancer incidence, such as chronic hepatitis or hepatic cirrhosis.

The composition of the present invention can be obtained by mixing or combining together carotenoid compounds and vitamin E compounds. Other ingredients may also be mixed or combined if these ingredients are to be included in the composition of the present invention.

### Carotenoid compounds

Carotenoids generally refer to pigments that are widely distributed among plants and animals, and any carotenoid compound can be used without limitations in the present invention. Examples of such carotenoids include hydrocarbon carotenoids such as carotene (α-carotene, β-carotene, γ-carotene, δ-carotene), lycopene, phytoene, phytofluene, and fucoxanthin; xanthophylls such as lutein, zeaxanthin, β-cryptoxanthin, lactucaxanthin, fucoxanthin, and astaxanthin; and the like.

A composition of the present invention comprising at least two types of carotenoids is particularly preferred. Examples of such combinations include combinations of α-carotene, β-carotene, and lycopene; combinations of α-carotene, β-carotene, and phytoene; combinations of α-carotene, lycopene, and phytoene; combinations of β-carotene, lycopene, and phytoene; combinations of α-carotene, β-carotene, lycopene, and phytoene; combinations of phytoene and lycopene; combinations of α-carotene and β-carotene; combinations of α-carotene and lycopene; combinations of α-carotene and phytoene; combinations of β-carotene and lycopene; combinations of β-carotene and phytoene; combinations of fucoxanthin and β-cryptoxanthin; combinations of fucoxanthin and lutein; combinations of β-cryptoxanthin and lutein; combinations of fucoxanthin, lutein, and β-cryptoxanthin; combinations of α-carotene, β-carotene, lycopene, and lutein; combinations of α-carotene, β-carotene, lycopene, and β-cryptoxanthin; combinations of α-carotene, β-carotene, lycopene, and fucoxanthin; and the like.

Above all, combinations of hydrocarbon carotenoids are preferable for targeting hepatic cancer or pulmonary cancer, and combinations of hydrocarbon carotenoids and xanthophyllic carotenoids are preferable for targeting colon cancer.

The carotenoids may consist of natural extracts from food products or the like, or may be chemically synthesized, although naturally extracted carotenoids are preferable for obtaining cancer-preventive effects. Naturally-derived carotenoids are extracted from food products using solvents that are permissible for use in food preparation, and can be purified from the resulting extracts by various types of chromatography, recrystallization, and the like. Because carotenoid-containing natural products usually contain multiple types of carotenoids, carotenoids that are extracted from natural products usually consist of carotenoid mixtures. For example, a carotene mixture extracted and purified from tomatoes comprises lycopene, β-carotene, phytoene, phytofluene, and the like, and is preferred for use as a carotenoid in the present invention. A palm fruit carotenoid mixture extracted and purified from oil-palm fruit comprises β-carotene, α-carotene, lycopene, and the like, and is also preferred for use as a carotenoid in the present invention. Synthetic carotenoids may also consist of derivatives designed to alter solubility or to achieve other purposes.

In any case, impurities such as food ingredients and the like may be contained in the carotenoid compounds. When impurities exist, it is sufficient that the carotenoid compounds are included in the prescribed quantities. It is preferable that the purity level or content ratio of the carotenoid compounds is at least 5% by weight, and particularly at least 15% by weight. If the content ratio is too low, it becomes necessary to take a large quantity of the composition as a whole in order to take the necessary daily quantity of carotenoid compounds, and the necessary daily quantity of carotenoid compounds thus becomes difficult to take. The content ratio range of the present invention eliminates such inconvenience.

### Vitamin E compounds

Vitamin E compounds include tocopherols such as α-tocopherol, β-tocopherol, γ-tocopherol, and δ-tocopherol; tocotrienols such as α-tocotrienol, β-tocotrienol, γ-tocotrienol, and δ-tocotrienol; and the like. These can be used alone or in combinations of at least two types. Particularly, inclusion of tocopherols and tocotrienols is preferable.

These also may consist of chemically synthesized products (dl) or naturally-derived products (d); however, naturally-derived products are preferable for obtaining cancer-preventive effects. Because natural products that contain vitamin E usually contain both tocopherols and tocotrienols, a vitamin E mixture extracted from natural products can be suitable for use in the present invention. Examples of vitamin E mixtures extracted and purified from natural products include products extracted from rice bran, palm oil, soybeans, corn, wheat germ, or the like with the aid of the aforementioned solvents permissible for use in food preparation. Vitamin E mixtures derived from rice bran or palm oil are particularly preferred. Synthetic vitamin E may also consist of derivatives designed to alter solubility or to achieve other purposes.

In any case, impurities such as food ingredients and the like may be included in the vitamin E compounds. When impurities exist, it is sufficient that the vitamin E compounds are included in the prescribed quantities. It is preferable that the purity level or content ratio of the vitamin E compounds is at least 30% by weight, and particularly at least 70% by weight. If the content ratio is too low, it becomes necessary to take a large quantity of the composition as a whole in order to take the necessary daily quantity of vitamin E compounds, and the necessary daily quantity of vitamin E compounds thus becomes difficult to take. This inconvenience does not occur within the content ratio range of the present invention.

### Other ingredients

The composition for human cancer prevention of the present invention may comprise naturally occurring antioxidant compounds, such as those shown below. These compounds may be extracted and purified from natural products, or may be chemically synthesized, although products that are extracted and purified from natural products are preferable for obtaining cancer-preventive effects. Derivatives thereof can also be used in chemically synthesized products.

In any case, impurities such as food ingredients and the like may be included in these compounds. When impurities exist, it is sufficient that the compounds are included in the prescribed quantities. It is preferable that the purity level or content ratio of each compound is at least 30% by weight, and particularly at least 70% by weight.

### <Flavonoid compounds>

The composition for human cancer prevention composition of the present invention may comprise flavonoid compounds in addition to carotenoid compounds and vitamin E compounds. Any type of flavonoid compound may be used. Hesperidin, nobiletin, luteolin, isoliquiritigenin, genistein, resveratrol, and the like are particularly preferred as flavonoid compounds, with hesperidin and nobiletin being particularly preferred. Either or both hesperidin and nobiletin are contained in the pulp or fruit of mandarin oranges, for example, and flavonoid mixtures extracted and purified from the pulp or fruit of mandarin oranges using solvents permissible for use in food preparation can be suitable for use in the present invention.

### <Ascorbic acid compounds>

The composition for human cancer prevention of the present invention may comprise ascorbic acid compounds in addition to carotenoid compounds and vitamin E compounds. Ascorbic acid compounds may also be contained in addition to carotenoid compounds, vitamin E compounds, and flavonoid compounds.

Naturally-derived L-ascorbic acid (vitamin C) is preferable as an ascorbic acid. L-ascorbic acid extracted and purified from lemons, acerola, or the like can, for example be used as a naturally-derived ascorbic acid. The ascorbic acid may also consist of derivatives designed to alter solubility or to achieve other purposes.

### <Other compounds>

Vitamin B2 tetrabutyrate, anthocyanin compounds, catechin, curcumin, ferulic acid, and the like can also be cited as antioxidant compounds. One or more types of these may be contained in the composition of the present invention.

The types of anthocyanin compounds are not particularly limited. Aglycon (anthocyanidin) from which sugar has been removed can also be used as an anthocyanin compound. Products extracted and purified from eggplant, blueberries, or the like can, for example, be used as naturally-derived anthocyanin compounds. Eggplant-derived delphinidin is particularly preferred.

Catechin extracted and purified from green tea, for example, can be used. Curcumin extracted and purified from turmeric roots, for example, can be used. Ferulic acid extracted and purified from rice bran, for example, can be used.

### Using proportions

Approximately 150-5000 parts by weight of vitamin E compounds per 100 parts by weight of carotenoid compounds is usually preferred for the composition of the present invention; approximately 150-1000 parts by weight of vitamin E compounds is particularly preferred; and approximately 200-400 parts by weight of vitamin E compounds is even more preferred.

Side effects can occur if the content level of vitamin E compounds is too high with respect to that of the carotenoid compounds, and the effects of the vitamin E cannot be obtained if the content level thereof is too low. This problem is obviated within the range of the present invention.

It is particularly preferable that the carotenoid compounds other than lycopene comprise total approximately 5-12 parts by weight (particularly about 10 parts by weight) per 10 parts by weight of lycopene; and that vitamin E compounds comprise approximately 30-100 parts by weight (particularly about 50 parts by weight) per 10 parts by weight of lycopene. It is even more preferable that approximately 3-6 parts by weight (particularly about 6 parts by weight) of β-carotene, approximately 1-3 parts by weight (particularly about 3 parts by weight) of α-carotene, approximately 1-3 parts by weight (particularly about 1 part by weight) of phytoene, and approximately 30-100 parts by weight (particularly about 50 parts by weight) of vitamin E compounds are contained per 10 parts by weight of lycopene.

When flavonoid compounds are added, it is preferable that flavonoid compounds comprise approximately 100-500 parts by weight (particularly about 100-250 parts by weight) per 100 parts by weight of carotenoid compounds. A bitter taste makes intake difficult if the content level of flavonoid compounds with respect to carotenoid compounds is too high, and the effects of the flavonoid compounds cannot be obtained if the content level thereof is too low. This problem is obviated within the range of the present invention.

When ascorbic acid compounds are contained, it is preferable that ascorbic acid compounds comprise approximately 100-1000 parts by weight (particularly about 100-250 parts by weight) per 100 parts by weight of carotenoid compounds. A sour taste makes intake difficult if the content level of ascorbic acid compounds with respect to carotenoid compounds is too high, and the effects of the ascorbic acid compounds cannot be obtained if the content level thereof is too low. This problem is obviated within the range of the present invention.

Also, when antioxidant compounds such as vitamin B2 tetrabutyrate, anthocyanin compounds, catechin, curcumin, ferulic acid, and the like are contained, it is preferable that each of these comprises approximately 5-250 parts by weight (particularly about 10-100 parts by weight) per 100 parts by weight of carotenoid compounds.

It is preferable that carotenoid compounds are contained in the composition of the present invention such that the daily dosage of carotenoid compounds is approximately 1-100 mg, and particularly 6-20 mg. It is also preferable that vitamin E compounds are included such that the daily dosage of vitamin E compounds is approximately 10-200 mg, and particularly about 30-100 mg. In other words, it is preferable, for example, that one-third of this amount is present in each dose when taken three times a day.

When the carotenoid compounds consist of lycopene and carotenoids other than lycopene, it is preferable that a daily dosage comprise approximately 5-15 mg (particularly about 10 mg) of lycopene and approximately 5-15 mg (particularly about 10 mg)of carotenoids other than lycopene, and that the total carotenoid quantity is adjusted to approximately 20 mg.

The amount of a daily dosage should more preferably comprise approximately 5-15 mg (particularly about 10 mg)of lycopene, approximately 3-6 mg (particularly about 6 mg)of β-carotene, approximately 3-6 mg (particularly about 3 mg)of α-carotene, approximately 1-3 mg (particularly about 1 mg)of phytoene, and approximately 30-100 mg (particularly about 50 mg)of vitamin E compounds.

When flavonoid compounds are included, the content thereof should preferably be such that a daily dosage comprises approximately 20-200 mg, and particularly about 25-50 mg, of the compounds. Also, when ascorbic acid compounds are included, the content thereof should preferably be such that a daily dosage comprises approximately 20-200 mg, and particularly about 20-50 mg, of the compounds. Quantities of other antioxidants should preferably be such that a daily dosage comprises approximately 1-50 mg, and particularly about 2-20 mg, of the compounds.

It is preferable that each carotenoid compound (the individual compounds in the carotenoid compounds) contained in the composition of the present invention is added such that the daily dosage thereof is approximately 1-20 mg. Cancer-preventive effects actually decline if too much of each carotenoid compound is contained, sometimes even inducing pulmonary cancer or the like instead. In contrast, if the content level of each compound is too low, adding the compound has no effect. This problem is obviated within the range of the present invention. It is extremely important to add appropriate quantities of antioxidant compounds (including vitamin E compounds), because daily intake of excessively large amounts of these products alone can also cause side effects.

### Pharmaceutical preparation

The composition of the present invention can be fashioned into a pharmaceutical preparation by the addition of ingredients used for drug preparations. The ingredients used for drug preparations may be conventional materials according to dosage forms.

Dosage forms are not particularly limited, and include capsulae, oils, granula, granula subtilae, pulveres, tabellae, pilulae, trochisci, or the like. Oils and oil-filled capsules are particularly preferred because of their lack of ingredient decomposition during manufacturing, and capsules are further preferable for being easy to take.

These pharmaceutical preparations may be prepared according to the common procedure as described in the United States Pharmacopoeia, for example. Tomato-derived oleoresin, mandarin orange pulp, or the like can be suitable for use as a vehicle or carrier.

The content level of carotenoid compounds and vitamin E compounds within the drug preparations are as previously described.

### Food product

The composition of the present invention can be fashioned into a food product by the addition of food products. Types of food products are not particularly limited, and include beverages such as teas, juices, and liquors; confections such as jellies and biscuits; processed food products such as rice and soft rice(or porridge); or the like.

It is preferable that the necessary daily dosage of the previously described groups of compounds or individual compounds is contained in amounts commensurate with the daily intake of food product when the food product of the present invention is involved. The concentrations of the groups of compounds or individual compounds should be uniformly distributed within the food product such that both the necessary daily dosage of the active ingredients and the food product can be easily taken.

### (II) Human Cancer Preventive Method

The human cancer preventive method of the present invention comprises a method for preventing cancer in humans by means of administering the previously described composition of the present invention.

Subjects are not particularly limited, and can be people with illness or people who are healthy. People with lifestyle habits that could lead to cancer, and people affected by diseases for which the probability of cancer incidence is high have a particularly high order of priority as subjects for the preventive method of the present invention. Furthermore, people who are likely to acquire familial cancers, and such people as those who are diagnosed with a risk of cancer by means of gene diagnoses based on single-nucleotide polymorphism or the like may also be targeted.

As previously mentioned, patients suffering from diseases likely to progress towards cancer, and people whose physical condition makes them susceptible to cancer are suitable subjects for the present invention. Examples of patients who can be targeted include patients of hepatic diseases likely to progress into hepatic cancer, including chronic hepatitis, hepatic cirrhosis, or the like, or hepatitis virus carriers; people having gene mutations which could lead to breast cancer; patients having familial colonic polyposis (a disease that could lead to colon cancer), patients having bronchial lesions which could lead to lung cancer; patients of UV dermatitis that could progress towards skin cancer; and similar patients. The preventive method of the present invention is most appropriate for targeting patients of hepatic diseases likely to progress into hepatic cancer, such as chronic hepatitis, hepatic cirrhosis, and the like.

It is preferable that the daily dosage of carotenoid compounds and vitamin E compounds is a quantity such that a daily dosage comprises approximately 1-100 mg (particularly about 6-20 mg) of carotenoid compounds and approximately 10-200 mg (particularly about 30-100 mg) of vitamin E compounds. In other words, it is preferable, for example, that one-third of a daily dosage is administered when the composition is taken three times a day.

When the carotenoid compounds consist of lycopene and carotenoids other than lycopene, it is preferable that a daily dosage comprise approximately 5-15 mg (particularly about 10 mg) of lycopene and approximately 5-15 mg (particularly about 10 mg)of carotenoids other than lycopene, and that the total carotenoid quantity is adjusted to approximately 20 mg.

The amount of a daily dose more preferably comprises approximately 5-15 mg (particularly about 10 mg)of lycopene, approximately 3-6 mg (particularly about 6 mg)of β-carotene, approximately 3-6 mg (particularly about 3 mg)of α-carotene, approximately 1-3 mg (particularly about 1 mg)of phytoene, and approximately 30-100 mg (particularly about 50 mg)of vitamin E compounds.

When flavonoid compounds are also administered, the daily dosage should preferably comprise about 20-200 mg, and particularly about 25-50 mg. When ascorbic acid compounds are also administered, the daily dosage should preferably comprise about 20-200 mg, and particularly about 20-50 mg. When other antioxidants are administered, the daily dosage should preferably comprise about 1-50 mg, and particularly about 2-20 mg.

A daily dosage of approximately 1-20 mg of each carotenoid compound (the individual compounds in the carotenoid compounds) is preferable. Cancer-preventive effects actually decline if too much of each carotenoid compound is contained, sometimes even inducing pulmonary cancer or the like instead. In contrast, if the content level of each compound is too low, adding the compound has no effect. This problem is obviated within the range of the present invention. It is extremely important to add appropriate quantities of antioxidant compounds (including vitamin E compounds), because daily intake of excessively large amounts of these products alone can also cause side effects.

When the daily dosage of certain compounds from among the active ingredients in the composition of the present invention has already been met or exceeded through the intake of food products, the remaining compounds constituting the composition of the present invention should be administered without the administration of the already taken compounds constituting the composition of the present invention. For example, if a patient has taken a sufficient quantity of carotenoid-rich tomatoes, no additional administration of carotenoid compounds from the composition of the present invention is needed.

About 1-4 administrations may be performed daily. Particularly, two administrations per day at intervals of about 6-12 hours are preferable. One administration per day is also preferable. Carotenoid compounds and vitamin E compounds may be administered simultaneously or separately. However, simultaneous administration thereof is preferable. When other ingredients are administered, such other ingredients may be administered simultaneously with carotenoid compounds, or may be administered separately. However, simultaneous administration thereof is preferable.

### EXAMPLES

A more detailed description of the present invention will be given hereafter using working examples and trial examples, but the present invention is not limited by these examples.

### Trial Examples

Capsules containing the ingredients below were administered to 46 human hepatic cirrhosis patients once a day for five years. The ingredients were encapsulated in their natural extract or synthetic compound form, with no granulation performed.

| | |
|---|---|
| Natural lycopene | 10 mg (tomato-derived) |
| Natural β-carotene | 6 mg (palm fruit, tomato-derived) |
| Natural α-carotene | 3 mg (palm fruit-derived) |
| Other natural carotenoids | 1 mg (palm fruit, |
| (mainly phytoene) | tomato-derived) |
| Synthetic α-tocopherol | 50 mg |

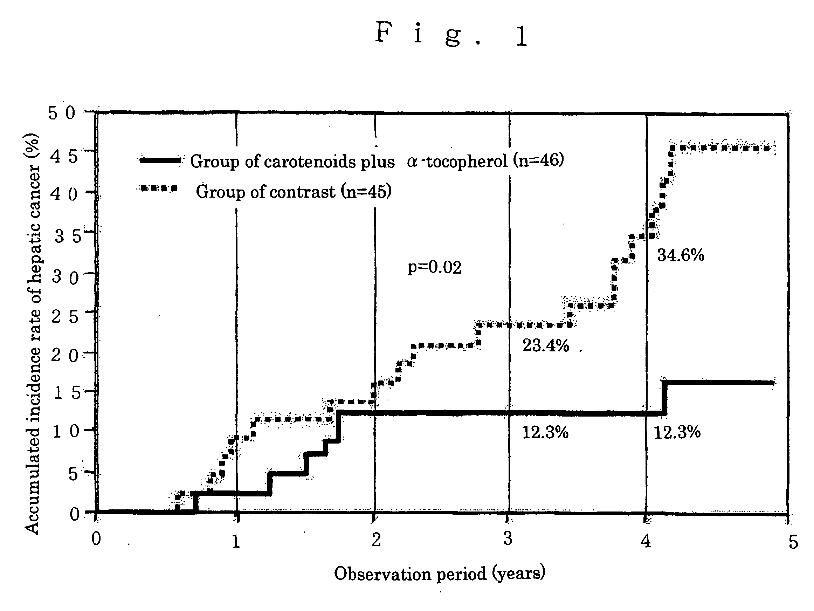

The incidence rate of hepatic cancer during the capsule intake period is shown by the graph in Fig. 1. The incidence rate of hepatic cancer for a contrast group of 45 human hepatic cirrhosis patients who were not taking the capsules is also shown. As shown in Fig. 1, no difference is noted between either group for 2 years after the first intake; however, the hepatic cancer incidence rate in the capsule group later declined, with the hepatic cancer incidence rate in the capsule group after 5 years reaching approximately one-third that of the non-capsule group. By taking the capsule of the present invention, it is apparent that the incidence of hepatic cancer is significantly suppressed after 3 years from the first intake.

Approximately one-third of the capsule group also experienced an alleviation of general malaise.

### Prescription Example

### <Example 1 (hepatic cancer preventive drug)>

### Composition of ingredients contained in 2 capsules (daily dosage):

| | |
|---|---|
| Lycopene | 10 mg (preferably |
| | tomato-derived) |
| Lutein | 6 mg (preferably spinach or |
| | marigold-derived) |
| β-Carotene | 2 mg (preferably palm |
| | fruit-derived) |
| α-Carotene | 1 mg (preferably palm |
| | fruit-derived) |
| Other carotenoids (mainly | 1 mg (preferably palm fruit, |
| phytoene) | tomato-derived) |
| Curcumin | 100 mg (preferably |
| | turmeric-derived) |
| Sylymarin | 10 mg (preferably lady's- |
| | thistle-derived) |
| Vitamin E mixture | 50 mg (preferably rice |
| | bran-derived) |
| Vitamin C | 100 mg (preferably lemon or |
| | acerola-derived) |
| Inositol | 200 mg (preferably rice |
| | bran-derived) |

### <Example 2 (breast cancer preventive drug)>

### Composition of ingredients contained in 2 capsules (daily dosage):

| | |
|---|---|
| Lycopene | 10 mg (preferably |
| | tomato-derived) |
| Phytoene | 10 mg (preferably a natural |
| | derivative) |
| Vitamin E mixture | 50 mg (preferably rice |
| | bran-derived) |

### <Example 3 (colon cancer preventive drug)>

### Composition of ingredients contained in 2 capsules (daily dosage):

| | |
|---|---|
| β-Cryptoxanthin | 10 mg (preferably mandarin |
| | orange-derived) |
| Lutein | 6 mg (preferably spinach or |
| | marigold-derived) |
| Fucoxanthin | 4 mg (preferably wakame or |
| | hiziki-derived) |
| Vitamin E mixture | 50 mg (preferably rice |
| | bran-derived) |

### <Example 4 (pulmonary cancer preventive drug)>

### Composition of ingredients contained in 2 capsules (daily dosage):

| | |
|---|---|
| Lutein | 10 mg (preferably spinach or |
| | marigold-derived) |
| β-Carotene | 6 mg (preferably palm |
| | fruit-derived) |
| α-Carotene | 3 mg (preferably palm |
| | fruit-derived) |
| Lycopene | 1 mg (preferably tomato-derived) |
| Vitamin B2 tetrabutyrate | 10 mg (synthetic compound) |
| Vitamin E mixture | 50 mg (preferably rice |
| | bran-derived) |

### <Example 5 (skin cancer preventive drug)>

### Composition of ingredients contained in 2 capsules (daily dosage):

| | |
|---|---|
| β-Cryptoxanthin | 10 mg (preferably mandarin |
| | orange-derived) |
| Fucoxanthin | 10 mg (preferably wakame or |
| | hiziki-derived) |
| Vitamin E mixture | 50 mg (preferably rice |
| | bran-derived) |

### <Example 6 (colon cancer preventive food product)>

### Composition of ingredients contained in 1 serving (daily intake):

| | |
|---|---|
| β-Cryptoxanthin | 10 mg (preferably mandarin |
| | orange-derived) |
| Fucoxanthin | 10 mg (preferably wakame or |
| | hiziki-derived) |
| Nobiletin | 10 mg (preferably citrus nobilis |
| | lour-derived) |
| Vitamin E mixture | 50 mg (preferably rice |
| | bran-derived) |
| Vitamin C | 100 mg (preferably lemon or |
| | acerola-derived) |
| Mandarin orange juice | 180 mL |

### <Example 7 (colon cancer preventive food product)>

### Composition of ingredients contained in 5 servings (daily intake):

| | |
|---|---|
| β-Cryptoxanthin | 10 mg (preferably mandarin |
| | orange-derived) |
| Fucoxanthin | 10 mg (preferably wakame or |
| | hiziki-derived) |
| Nobiletin | 10 mg (preferably flat |
| | lemon-derived) |
| Vitamin E mixture | 50 mg (preferably rice |
| | bran-derived) |
| Vitamin C | 100 mg |
| Jelly | 30 g |

### INDUSTRIAL APPLICABILITY

Cancer-preventive effects in humans are manifested for the first time in the composition of the present invention. Therefore, the progression towards cancer is suppressed when the composition of the present invention is administered in humans, especially for people who are suffering from diseases that can progress towards cancer. Particularly, the progression towards hepatic cancer in patients of hepatic diseases for which there is a potential for progression towards hepatic cancer (chronic hepatitis, hepatic cirrhosis, and the like) is effectively suppressed.

Also, general malaise is alleviated and QOL (Quality of Life) is improved when the composition of the present invention is administered to such patients.

The composition of the present invention having the effects described above may be used in the form of pharmaceutical preparations or food products. Namely, human cancer can be effectively prevented by administration of pharmaceutical embodiments or food product embodiments to humans, in addition to embodiments involving active ingredients alone.

### BRIEF DESCRIPTION OF THE DRAWINGS

Fig. 1 is a graph showing the human hepatic cancer preventive effect in an example of the method of the present invention.

## Claims

1. A composition comprising carotenoid compounds and vitamin E compounds.

2. The composition according to claim 1, comprising at least two types of carotenoid compounds.

3. The composition according to claim 1, comprising lycopene and carotenes as carotenoid compounds.

4. The composition according to claim 1, comprising 150-5000 parts by weight of vitamin E compounds per 100 parts by weight of carotenoid compounds.

5. The composition according to claim 1, comprising 10 parts by weight of lycopene, 3-6 parts by weight of β-carotene, 1-3 parts by weight of α-carotene, 1-3 parts by weight of phytoene, and 30-100 parts by weight of vitamin E compounds.

6. The composition according to claim 5, comprising 10 parts by weight of lycopene, 6 parts by weight of β-carotene, 3 parts by weight of α-carotene, 1 part by weight of phytoene, and 50 parts by weight of vitamin E compounds.

7. The composition according to claim 1, further comprising flavonoid compounds.

8. The composition according to claim 1, further comprising ascorbic acid compounds.

9. The composition according to claim 1, further comprising at least one antioxidant compound selected from a group consisting of vitamin B2 tetrabutyrate, anthocyanin compounds, catechin, curcumin, inositol, and ferulic acid.

10. The composition according to claim 1, comprising carotenoid compounds and vitamin E compounds such that a daily dosage consists of 1-100 mg of carotenoid compounds and 10-200 mg of vitamin E compounds.

11. The composition according to claim 1, in the form of a pharmaceutical preparation.

12. The composition according to claim 1, further comprising food products.

13. A method of human cancer prevention for preventing cancer in humans by means of administering_a composition that comprises carotenoid compounds and vitamin E compounds.

14. The method of human cancer prevention according to claim 13, wherein the composition comprises at least two types of carotenoid compounds.

15. The method of human cancer prevention according to claim 13, wherein the composition comprises lycopene and carotenes as carotenoid compounds.

16. The method of human cancer prevention according to claim 13, wherein the composition comprises 150-5000 parts by weight of vitamin E compounds per 100 parts by weight of carotenoid compounds.

17. The method of human cancer prevention according to claim 13, wherein the composition comprises 10 parts by weight of lycopene, 3-6 parts by weight of β-carotene, 1-3 parts by weight of α-carotene, 1-3 parts by weight of phytoene, and 30-100 parts by weight of vitamin E compounds.

18. The method of human cancer prevention according to claim 17, wherein the composition comprises 10 parts by weight of lycopene, 6 parts by weight of β-carotene, 3 parts by weight of α-carotene, 1 part by weight of phytoene, and 50 parts by weight of vitamin E compounds.

19. The method of human cancer prevention according to claim 13, wherein the composition further comprises flavonoid compounds.

20. The method of human cancer prevention according to claim 13, wherein the composition further comprises ascorbic acid compounds.

21. The method of human cancer prevention according to claim 13, wherein the composition further comprises at least one antioxidant compound selected from a group consisting of vitamin B2 tetrabutyrate, anthocyanin compounds, catechin, curcumin, inositol, and ferulic acid.

22. The method of human cancer prevention according to claim 13, wherein the composition is in the form of a pharmaceutical preparation.

23. The method of human cancer prevention according to claim 13, wherein the composition further comprises food products.

24. The method of human cancer prevention according to claim 13, wherein 1-100 mg a day of carotenoid compounds and 10-200 mg a day of vitamin E compounds are administered.

25. The method of human cancer prevention according to claim 24, wherein 5-15 mg a day of lycopene, 1-10 mg a day of β-carotene, 1-10 mg a day of α-carotene, 1-10 mg a day of phytoene, and 10-200 mg a day of vitamin E compounds are administered.

26. The method of human cancer prevention according to claim 13, wherein the daily dosage of a single carotenoid compound is no more than 20 mg.

27. The method of human cancer prevention according to claim 13, for preventing the progression towards hepatic cancer from hepatic diseases in human.
